# EUROPEAN PATENT SPECIFICATION

(11) **EP 2 320 912 B1**
(45) Date of publication and mention of the grant of the patent: **14.01.2015**
(21) Application number: 09787409.3
(22) Date of filing: 05.08.2009
(51) Int. Cl.: A61K 31/56, A61P 27/06, A61P 27/02

(54) **TREATMENT OF RETINAL DEGENERATION**
BEHANDLUNG VON NETZHAUTDEGENERATION
TRAITEMENT DE DÉGÉNÉRESCENCE DE LA RÉTINE

(30) Priority: 05.08.2008 IE 20080633
(43) Date of publication of application: 18.05.2011
(73) Proprietor: University College Cork-National University of Ireland, Cork, Cork (IE)
(72) Inventor: COTTER, Thomas, Co. Cork (IE); DOONAN, Francesca, Co. Cork (IE); CLERKIN, Sebastian, Cork (IE); O'DRISCOLL Carolyn, Cork (IE)
(74) Representative: Purdylucey Intellectual Property
(86) International application number: PCT/IE2009/000053
(87) International publication number: WO 2010/016042

(56) References cited:
- WO-A-2006/082588
- WO-A-2008/070726
- WO-A-2008/150547
- US-A- 3 959 322
- US-A1- 2006 205 704
- SATHASIVAM S ET AL: "Apoptosis in amyotrophic lateral sclerosis-what is the evidence?" LANCET NEUROLOGY, LANCET PUBLISHING GROUP, LONDON, GB, vol. 4, no. 8, 1 August 2005 (2005-08-01), pages 500-509, XP004982687 ISSN: 1474-4422
- CORDEIRO M FRANCESCA ET AL: "Real-time imaging of single nerve cell apoptosis in retinal neurodegeneration", PROCEEDINGS OF THE NATIONAL ACADEMY OF SCIENCES OF THE UNITED STATES OF AMERICA, vol. 101, no. 36, 7 September 2004 (2004-09-07), pages 13352-13356, ISSN: 0027-8424
- DATABASE BIOSIS [Online] BIOSCIENCES INFORMATION SERVICE, PHILADELPHIA, PA, US November 2002 DUNAIEF JOSHUA L ET AL: 'The role of apoptosis in age-related macular degeneration.' Database accession no. PREV200300010857 & DUNAIEF JOSHUA L ET AL: "The role of apoptosis in age-related macular degeneration.", ARCHIVES OF OPHTHALMOLOGY, vol. 120, no. 11, November 2002 (2002-11), pages 1435-1442, ISSN: 0003-9950

## Description

### TECHNICAL FIELD

The invention relates to a method of treating or preventing a disease or condition characterised by apoptosis or degeneration of mammalian cells, especially retinal photoreceptive cells.

### BACKGROUND TO THE INVENTION

The loss of retinal cells in Age-related Macular Degeneration (AMD) and Glaucoma are the two leading causes of blindness in the developed worked. Retinitis Pigmentosa (RP) is a rarer related condition that also leads to loss of sight. RP is a group of hereditary disorders of the retina caused by mutations in numerous genes involved in photoreceptor structure or function. The disease is characterized by early loss of photoreceptors leading to blindness. Glaucoma is caused by a number of different pathological mechanisms that in most cases result in elevated intraocular pressure (IOP) within the eye. Like RP, it is a multiple gene-related disease and genetic factors play a complex role in glaucoma predisposition. Over time, the increase in IOP causes damage to the optic nerve and gradual and continuous loss of retinal ganglion cells. Cell loss in AMD (Age-related Macular Degeneration) also occurs as a result of apoptosis of retinal pigment epithelial (RPE) cells followed by apoptosis of photoreceptors. A central feature of each of the above diseases is that retinal cell loss occurs by a cell death process known as apoptosis.

There are no drugs on the market for the treatment of RP. There are a small number in early stage development, but most of them rely on repeated intavitreal injections directly into the eye for their effect or a gene therapy approach. For RP, there is a market realisation that preventing apoptosis may be a treatment option. In the case of glaucoma there are several treatment options currently available for this very large market segment. Most of these rely on the delivery of the drug in the form of eye drops. The goal of such treatments is to reduce the intra ocular pressure that is a causative factor that leads to the loss of retinal ganglion cells. Like RP there are no treatments focused on the prevention of retinal ganglion cell apoptosis. Finally, for AMD of which there are two main forms there are a number of treatment options ranging from laser therapy to the use of inhibitors that prevent blood vessel proliferation which is a characteristic of the condition and leads to loss of photoreceptors cells. Again each of these treatment options in invasive and requires repeated hospital visits. WO 2008/070726 describes the use of a propestapen, for example norgestrel, for the treatment of a dry eye condition. WHO 2006/082588 discloses the use of a mist of a pharmaceutical composition comprising protein or peptide active agents for ophthalmic delivery.

It is an object of the invention to overcome at least one of the above problems.

### BRIEF SUMMARY OF THE INVENTION

Accordingly, the invention relates to a composition for use in treating or preventing a retinal dystrophy.

Suitably, the compound, or Active, is a 13-polycarbon-alkyl-17-alkyl-17β-hydroxygon-4-en-3-one. Preferably, the Active is NORGESTREL^{™} (13β, 17α-diethyl-17β-hydroxygon-4-en-3-one).

Examples of these Actives, and their methods of manufacture are described in US Patent Serial Number 3,959,322 Specifically, the manufacture of NORGESTREL^{™} is described in Example 143 of the above-referenced Patent.

Suitably, the disease or condition is a retinal dystrophy, such as, for example, Retinitis Pigmentosa (RP), Glaucoma, or Age-related Macular Degeneration (AMD).

The invention also relates to the use of the Active as a medicament formulated for local delivery to the eye. Suitably, the medicament is for treating a retinal dystrophy, especially Retinitis Pigmentosa (RP), Glaucoma, or Age-related Macular Degeneration (AMD).

The invention also relates to a pharmaceutical composition formulated as a solution suitable for local delivery to the eye, the composition comprising the Active and a pharmaceutically acceptable carrier. Suitably, the pharmaceutical formulation is provided in a form selected from the group consisting of: an eye-drop solution; a solution suitable for intraocular injection; and a solution suitable for intravitreal injection.

### BRIEF DESCRIPTION OF THE FIGURES

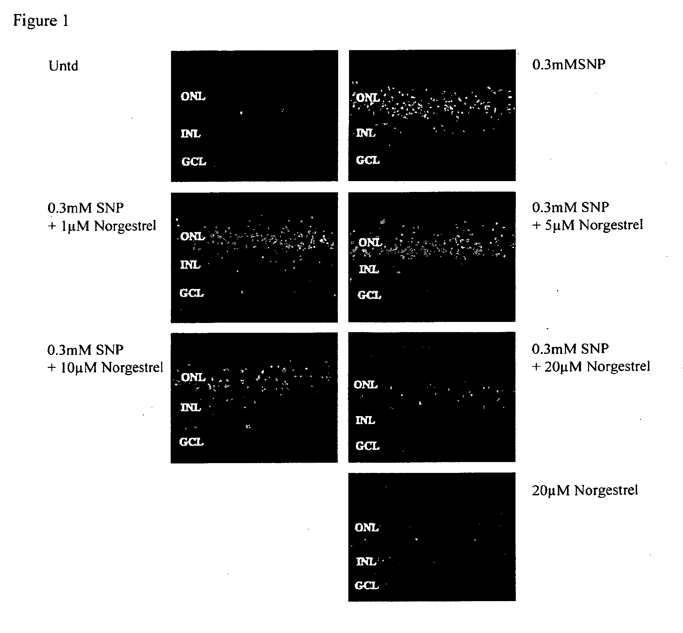

**Figure 1****:** This figures shows that SNP induces apoptosis at 24 hours in the outer nuclear layer (ONL) which contains the photoreceptor cells. Photoreceptors are protected from SNP induced apoptosis by increasing concentrations of norgestrel. The final panel shows that norgestrel at the highest concentration used is not toxic to retinal cells.

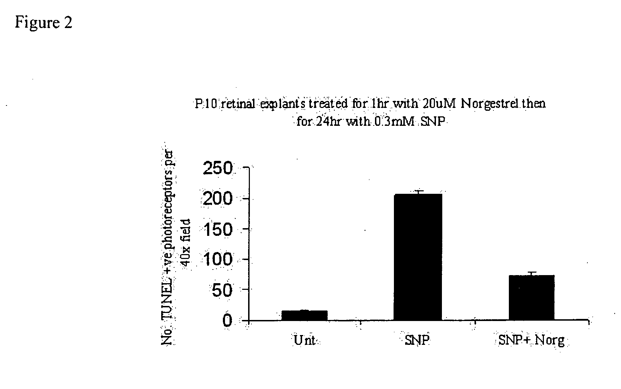

**Figure 2****:** This figure represents the results of 2 independent experiments. The number of TUNEL positive photoreceptor cells in 3 40x fields per sample was counted and graphed with error bars.

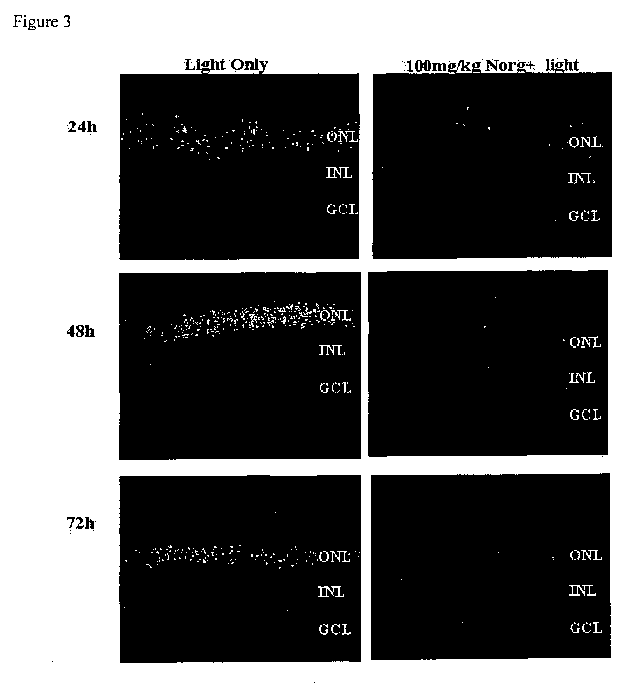

**Figure 3****:** In this figure the study was extended from *ex vivo* cultures to the light damage model *in vivo.* In this acute model of retinal disease albino balb/c mice are exposed to excessive white light causing the photoreceptor cells to die by apoptosis. This figure shows that mice exposed to bright white light undergo extensive apoptosis at 24, 48 and 72 hours post light damage. 1 hour pre-treatment with 100mg/kg norgestrel protects from the retinal damage observed. The protection extends to 72 hours with only a single injection of norgestrel.

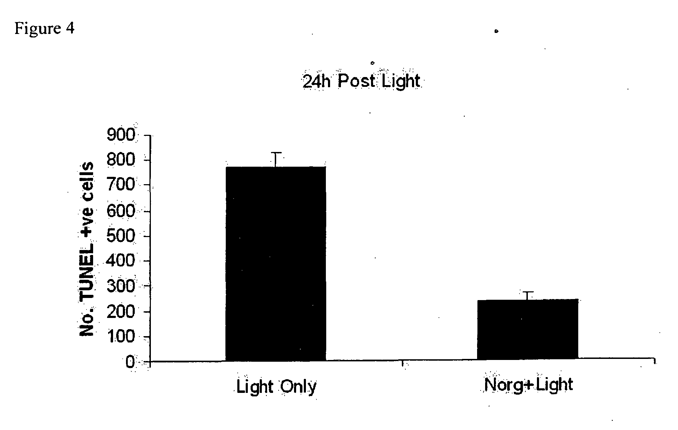

**Figure 4****:** A histogram of the data from Figure 3. TUNEL positive cells in three independent retinae were counted. Counts were performed on the central 40x field of the ONL and graphed with error bars (+/- standard deviation-SD). The graph indicates approx 70% protection from light induced cell death in the central retina at 24 hours. Only the 24 hour time point is shown here.

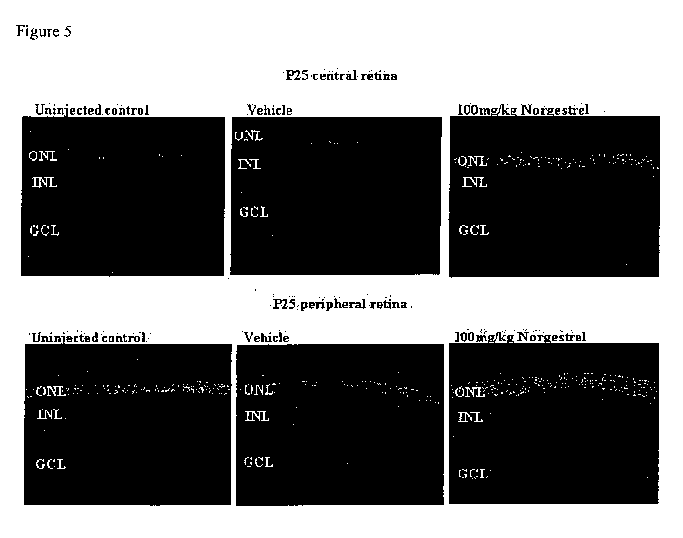

Figure 5 shows the results obtained in a chronic model of retinal degeneration called the rd10 model. In this model photoreceptor cells degenerate more slowly than the light model over weeks rather than hours to days. Cell death is evident from postnatal day 18 (P18) and by P25 a significant loss of photoreceptor cell layers is observed. Figure 5 shows protection of photoreceptor cells in the outer nuclear layer (ONL) when mice are injected daily with 100mg/kg norgestrel. The central and peripheral retina degenerate at different rates in the rd10 model but these data show that both areas of the retina are protected. The upper panel of images, taken from the central retina, show that there is a protection of 3-4 layers of photoreceptor nuclei in the ONL in response to norgestrel. The lower panel of images, taken from the peripheral retina, also show a protection of approx 3-4 layers in the ONL.

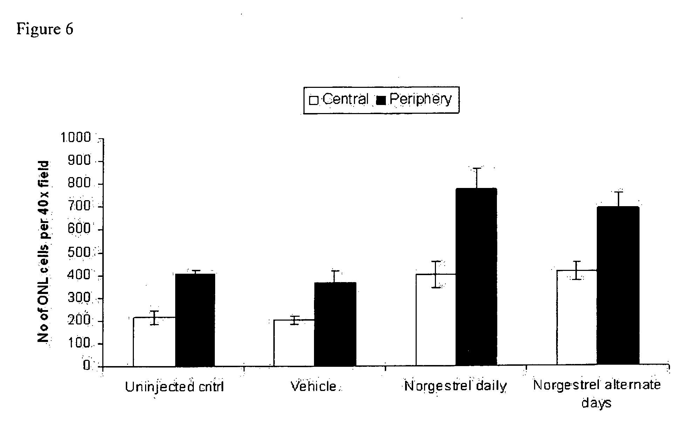

**Figure 6****:** Once it was established that daily injection of norgestrel protected from retinal degeneration in the rd10 model the dosing regimen was examined, To this end the injection frequency was reduced from daily to every second day. Figure 6 is a histogram of the data obtained. It shows that mice injected every second day are equally as protected as mice injected daily. For all figures hereafter mice have been injected every second day.

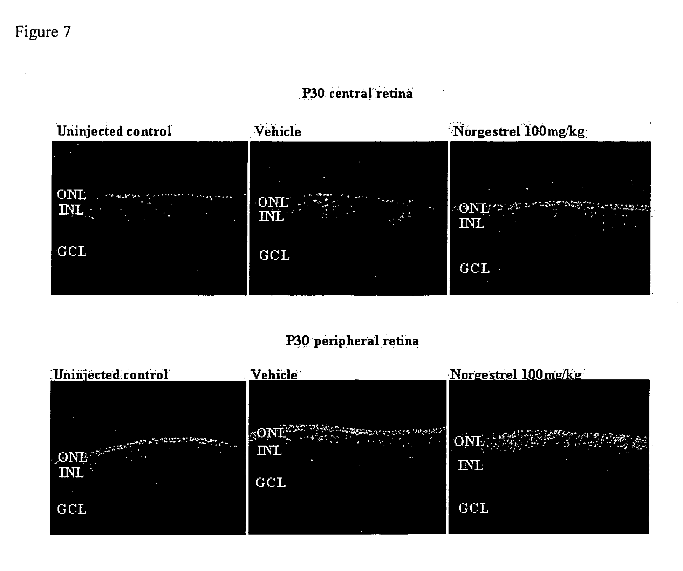

**Figure 7** extends the study in the rd10 model from P25 to P30, to determine whether photoreceptor cells continue to be protected. Intraperitoneal (IP) injection of 100mg/kg norgestrel in the rd10 mouse every second day from P18-P30 continues to protect photoreceptor cells in the ONL. The upper panel of images, taken from the central retina, show that there is a protection of 1-2 layers of photoreceptor nuclei in response to norgestrel. The lower panel of images, taken from the peripheral retina, show that the protection is much more significant in this region of the retina. A protection of approx 4-5 layers is achieved with norgestrel injection.

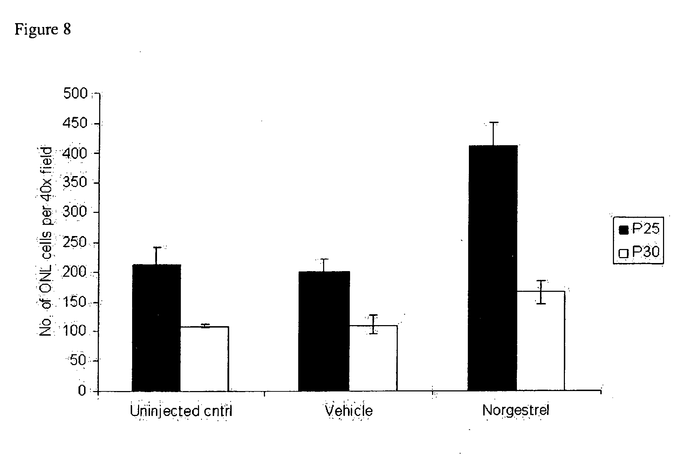

**Figure 8** compares the number of photoreceptor nuclei remaining in the central retina at P25 and P30 in vehicle versus norgestrel treated retinae using the data from figure 6 (time point: P25) and figure 7 (time point: P30). The central retina degenerates more slowly in norgestrel treated versus vehicle but the protection is more significant at P25 than at P30.

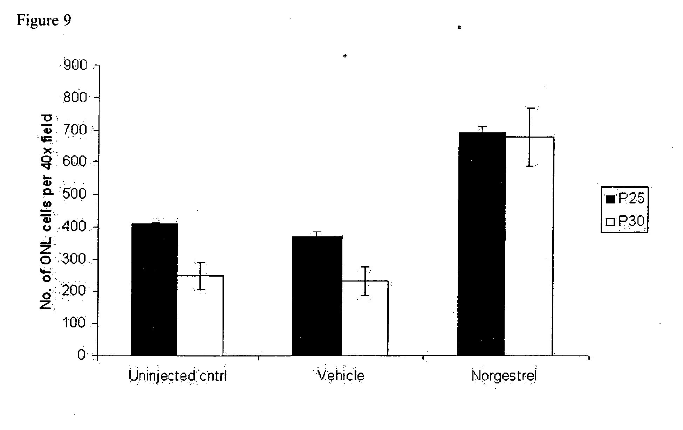

**Figure 9** also shows that the peripheral retina degenerates more slowly in norgestrel treated versus vehicle treated retinae. However, while the central does continue to degenerate from P25 to P30 albeit at a slower rate in norgestrel treated mice (figure 8), the degeneration of the peripheral retina virtually ceases. Very few cells are lost in the peripheral retinae of norgestrel treated mice between the ages of P25 and P30. This could be very significant if there is no further loss of photoreceptors in the periphery at even longer timepoints. This idea is currently undergoing further investigation.

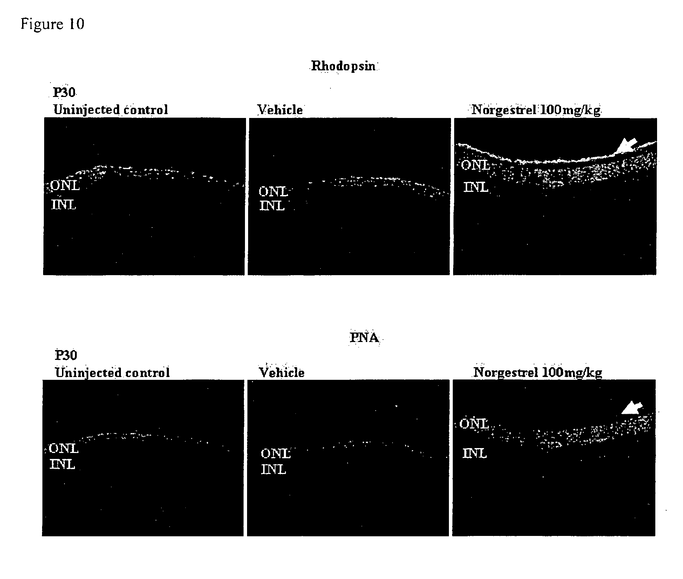

**Figure 10****:** The ONL comprises two types of cell; rod photoreceptors and cone photoreceptors. In figure 10 we identify which cell types are protected by norgestrel treatment. Rhodopsin is a protein specifically found in the outer segments of rod photoreceptors. Immunofluorescent staining using an antibody specifc to rhodopsin indicates that more rhodopsin positive cells remain in the ONL of norgestrel treated mice compared to control. The arrow in the upper third panel indicates the intensity of rhodopsin staining following norgestrel injection. This intense staining is clearly lacking in uninjected or vehicle injected controls. Peanut agglutinin (PNA) is a lectin which binds to carbohydrates found in the outer membrane of cone photoreceptors but which are absent from rods. Staining retinal sections with PNA indicates that more cone cells are found in the ONL following norgestrel treatment. The arrow in the lower third panel indicates the intensity of PNA staining following norgestrel injection. This intense staining is not evident in uninjected or vehicle injected controls. Therefore the cells of the ONL involved in both colour and black/white vision are protected in the rd10 model by norgestrel. The data presented herein demonstrates the efficacious nature of norgestrel in two physiologically relevant models of retinal degeneration: the acute light model and the more slowly progressive rd10 model. In the latter disease state norgestrel protects both rod and cone photoreceptor cell types and proves significantly efficacious in the peripheral retina, the area of the retina degenerating slowest in this model. The capacity of norgestrel to protect different cell types degenerating at different rates is very significant and the neuroprotective effect of norgestrel on a more distal retinal neuron, the ganglion cell, is currently under investigation in a mouse model of glaucoma.

### DETAILED DESCRIPTION OF THE INVENTION

The therapeutic product of the invention are directed against a retinal dystrophy.

In a preferred embodiment of the invention, the retinal degenerative condition is selected from the group comprising: RP; Glaucoma; retinopathies; and AMD.

In this specification the term "amount effective" should be taken to mean an amount which results in a clinically significant reduction of degeneration or apoptosis in cells having a phenotype characteristic of a degenerative condition (i.e. retinal photoreceptor cells from a patient with AMD or RP). Suitably, the Active is administered at a dose of between 1 microgram and 10 miligrams per ml, preferably between 10 micrograms and 5 miligrams per ml, more preferably between 100 micrograms and 2 miligrams per ml. Typically, it is given as a bolus dose. However, when continuous infusion is used, such as by intrathecal pump, the Active may be administed at a dosage rate of between 5 and 20 µg/kg/minute, preferably between 7 and 15 µg/kg/minute. In one embodiment of the invention, the Active is administered using a dosage regime which is similar to that employed when the medicament is administered as a female contraceptive. In the context of the therapeutic aspects of the present invention, the term "individual in need thereof" shall be taken to mean an individual who is afflicted with a disease or condition which involves apoptosis or degeneration of mammalian cells, especially apoptosis or degeneration of retinal photoreceptive cells. Retinal degenerative conditions or diseases such as RP, Glaucoma, Retinopathies, and AMD, are examples of such diseases or conditions.

In one embodiment of the invention, an individual in treated with the Active by direct delivery of the Active by a means selected from the group: intravenous delivery; intrperitoneal delivery; oral delivery; intramuscular delivery; intrathecal delivery; and inhaled delivery. Methods for achieving these means of delivery will be well known to those skilled in the art of drug delivery. Specific examples are provided below:
- Delivered intrathecially by mini-osmotoc pump. (ref: Ignacio et al., Ann. N.Y. Acad. Sci. 2005, 1053: 121-136).
- Intramuscular- delivery directly into muscle(s) by syringe or mini osmotic pump (Azzouz et al., Nat Med. 2005;11(4):429-33).
- Intraperitoneal- for systemic administration. Directly administered to peritoneum by syringe or mini osmotic pump (Kieran et al., Nat Med 2004; 10(4):402).
- Subcutaneous- for systemic administration. Directly administered below the skin by syringe (Reinholz et al., Exp Neurol. 1999;159(1):204-16).
- Intraventricular- direct administration to the ventricles in the brain, by injection or using small catheter attached to an osmotic pump.(Sathasivam et al., 2005 Neuropath App Neurobiol; 31(5): 467)
- Implant- Active can be prepared in an implant (eg small silicon implant) that will release the active. Implant can be placed at muscles or directly onto the spinal cord (Kieran and Greensmith, 2004 Neurosci 125(2):427-39).

Generally, when the individual is male, or a pre-menopausal female, the Active will be administered locally. When the individual is a post-menopausal female, the Active does not need to be administered locally.

In one embodiment of the therapy of the invention, the Active is linked to a coupling partner, e.g. an effector molecule, a label, a drug, a toxin and/or a carrier or transport molecule. Techniques for coupling the Active of the invention to both peptidyl and non-peptidyl coupling partners are well known in the art.

The invention provides use of a compound in the treatment and prevention of diseases or conditions characterized by apoptosis or degeneration of retinal photoreceptive cells, by administration to a subject in need of such treatment of a therapeutically or prophylactically effective amount of the Active. The subject is preferably an animal, including, but not limited to, animals such as monkeys, cows, pigs, horses, chickens, cats, dogs, etc., and is preferably a mammal, and most preferably human.

Apart from the specific delivery systems embodied below, various delivery systems are known and can be used to administer the Active of the invention, e.g., encapsulation in liposomes, microparticles, microcapsules. Methods of introduction include but are not limited to intradermal, intramuscular, intraperitoneal, intravenous, subcutaneous, intranasal, epidural, and oral routes. The Active may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the Active of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the Active of the invention locally to the area in need of treatment; this may be achieved, for example, by means of eye drops, intraocular injection, or an implant, said implant being of a porous, non-porous, or gelatinous material, including membranes, such as sialastic membranes, or fibers.

In another embodiment, the Active can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the Active can be delivered in a controlled release system. In one embodiment, a pump may be used (see Langer, supra; Sefton, CRC Crit. Ref. Biomed., Eng. 14:201 (1987); Buchwald et al., Surgery 88:75 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, thus requiring only a fraction of the systemic dose (see, e.g., Goodson, in Medical Applications of Controlled Release, supra, vol. 2, pp. 115-138 (1984)). Other controlled release systems are discussed in the review by Langer (Science 249:1527-1533 (1990)).

The present invention also provides pharmaceutical compositions comprising the Active. Such compositions comprise a therapeutically effective amount of the Active, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the Active is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The Active of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the Active of the invention which will be effective in the treatment of a particular disorder or condition will depend on the nature of the disorder or condition, and can be determined by standard clinical techniques. In addition, in vivo and/or in vitro assays may optionally be employed to help predict optimal dosage ranges. The precise dose to be employed in the formulation will also depend on the route of administration, and the seriousness of the disease or disorder, and should be decided according to the judgment of the practitioner and each patient's circumstances.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### Exnerimental

### Ex-vivo Methods

**Retinal explant culture:** Eyes from postnatal day 10, C57BL/6 mice were removed and cleaned with 70% ethanol. The anterior segment, vitreous body, and sclera were removed and the retina mounted on Millicell nitrocellulose inserts (Millipore, Billerica, MA) photoreceptor-side down. Explants were cultured without retinal pigment epithelium (RPE) in 1.2 ml of R16 specialised media (from Dr. P. A. Ekstrom, Wallenberg Retina Centre, Lund University, Lund, Sweden) without additional serum. Treated explants were cultured in medium containing 300µM of the nitric oxide donor SNP (sodium nitroprusside) for 24 h. Pre-treatment with norgestrel was for 1 hour. Figure 1 shows that photoreceptors are protected from SNP induced apoptosis by increasing concentrations of norgestrel. The final panel in Figure 1 shows that norgestrel at the highest concentration used in not toxic to retinal cells.

**Apoptosis detection by Terminal deoxynucleotidyl transferase-mediated biotinylated UTP nick end labeling (TUNEL):** Retinal explants were fixed in 10% neutral buffered formalin overnight at 4°C, followed by cryoprotection in 25% sucrose overnight at 4°C. Frozen sections (7µm) were incubated with terminal deoxynucleotidyl transferase (MSC, Dublin, Republic of Ireland) and fluorescein-12-dUTP (Roche, Lewes, UK) according to manufacturers' instructions at 37°C for 1h. Sections were mounted and viewed under a fluorescence microscope (Leica DM LB2; Leica, Nussloch, Germany) using an FITC filter. Figure 2 shows that norgestrel protects photoreceptive cells from SNP induced cell death in an ex-vivo retinal explant model.

**Peanut agglutinin (PNA) staining:** Eyes were fixed in 10% neutral buffered formalin overnight at 4°C, followed by cryoprotection in 25% sucrose overnight at 4°C. Frozen sections (7µm) were blocked with 0.1% bovine serum albumin (BSA) in 0.1% tween/PBS for 30 minutes at room temperature. Sections were incubated with rhodamine conjugated PNA (Invitrogen, Dun Laoghaire, Ireland) for 20 minutes at room temperature as per manufacturers' instructions. Sections were mounted and viewed under a fluorescence microscope (Leica DM LB2; Leica, Nussloch, Germany) using a TRITC filter.

**Immunofluorescence:** Eyes were fixed in 10% neutral buffered formalin overnight at 4°C, followed by cryoprotection in 25% sucrose overnight at 4°C. Frozen sections (7µm) were blocked with 0.1 % bovine serum albumin (BSA) in 0.1 % tween/PBS for 1 hour at room temperature. Sections were incubated with anti- rhodopsin antibody (LAB VISION Corporation, Fremont, CA., USA) overnight at 4°C. Sections were washed and incubated with FITC conjugated secondary mouse antibody (Dako, Glostrup, Denmark) for 1 hour at room temperature. Following further washes, sections were mounted and viewed under a fluorescence microscope (Leica DM LB2; Leica, Nussloch, Germany) using a FITC filter.

### In-vivo Methods

**Light damage model:** Balb/c mice were dark adapted for 18 h prior to exposure to constant light. Mice were injected intraperitoneally with 100mg/kg of norgestrel 1 hour prior to light damage. Immediately prior to light exposure their pupils were dilated with 0.5% cyclopentolate under red light. Retinal light damage was induced by exposure to 2 h of cool white fluorescent light at an illumination of 5000 lux. Following exposure to constant light, animals were placed in the dark for 24 h then killed immediately by cervical dislocation. Figure 3 shows that 2 hrs light damage induces apoptosis after 24 hours in the ONL. Photoreceptors are protected by IP injection of 100mg/kg norgestrel.
TUNEL staining was performed as described above.

**Rd10 model**: The rd10 mouse strain exhibits autosomal recessive retinal degeneration and has a point mumtion in exon 13 the Pde6b gene. It is a better model of the slow progression of typical human autosomalre recessive RP than the acute light model as photoreceptor cells are lost over a period of weeks rather than days. Loss of photoreceptors in the rd10 mouse begins at approximately 2 weeks of age, with the peak of photoreceptor death occurring at postnatal day (P) 25.

## Claims

1. A compound for use in the treatment or prevention of a retinal dystrophy, wherein the compound is a 13-polycarbon-alkyl-17-alkyl-17β-hydroxygon-4-en-3-one, or a pharmaceutically acceptable salt thereof.

2. The compound according to Claim 1 for the use of Claim 1 in which the retinal dystrophy is selected from the group consisting of: Retinitis Pigmentosa (RP); Glaucoma; or Age-related Macular Degeneration (AMD).

3. The compound according to Claim 1 or 2 for the use of Claim 1 or 2 in which the compound is 13β, 17α-diethyl-17β-hydroxygon-4-en-3-one, or a pharmaceutically acceptable salt thereof.

4. The compound according to any preceding Claim for use of any of the preceding claims in which the compound is locally administered to the eye.

5. The compound according to Claim 4 for the use of Claim 4 in which the compound is administered by means of application of eye drops, intravitreal injection or intraocular injection.

6. A pharmaceutical composition formulated as a solution for use in the treatment of a retinal dystrophy suitable for local delivery to the eye by intraocular injection and/or intravitreal injection, the composition comprising a pharmaceutically acceptable carrier and a 13-polycarbon-alkyl-17-alkyl-17β-hydroxygon-4-en-3-one compound, wherein the compound is 13β, 17α-diethyl-17β-hydroxygon-4-en-3-one.

## Patentansprüche

1. Verbindung für die Verwendung bei der Behandlung oder Prävention einer Netzhautdystrophie, worin die Verbindung ein 13-Polykohlenstoffalkyl-17-alkyl-17β-hydroxy-gon-4-en-3-on ist, oder ein pharmazeutisch verträgliches Salz davon.

2. Verbindung nach Anspruch 1 für die Verwendung nach Anspruch 1, in der die Netzhautdystrophie aus der Gruppe ausgewählt ist, die aus Folgenden besteht: Retinitis pigmentosa (RP); Glaukom; oder altersbedingte Makuladegeneration (AMD).

3. Verbindung nach Anspruch 1 oder 2 für die Verwendung nach Anspruch 1 oder 2, in der die Verbindung 13β,17α-Diethyl-17β-hydroxy-gon-4-en-3-on ist, oder ein pharmazeutisch verträgliches Salz davon.

4. Verbindung nach einem vorstehenden Anspruch für die Verwendung nach einem der vorstehenden Ansprüche, in der die Verbindung lokal an das Auge verabreicht wird.

5. Verbindung nach Anspruch 4 für die Verwendung nach Anspruch 4, in der die Verbindung durch Applikation von Augentropfen, intravitreale Injektion oder intraokulare Injektion verabreicht wird.

6. Pharmazeutische Zusammensetzung, die als Lösung für die Verwendung bei der Behandlung einer Netzhautdystrophie formuliert ist, die zur lokalen Abgabe an das Auge durch intraokulare Injektion und/oder intravitreale Injektion geeignet ist, wobei die Zusammensetzung einen pharmazeutisch verträglichen Träger und eine 13-Polykohlenstoffalkyl-17-alkyl-17β-hydroxy-gon-4-en-3-on-Verbindung umfasst, worin die Verbindung 13β,17α-Diethyl-17β-hydroxy-gon-4-en-3-on ist.

## Revendications

1. Composé pour une utilisation dans le traitement ou la prévention d'une dystrophie rétinienne, ledit composé étant une 13-polycarbon-alkyl-17-alkyl-17β-hydroxygon-4-èn-3-one ou un sel pharmaceutiquement acceptable de celle-ci.

2. Composé selon la revendication 1 pour l'utilisation de la revendication 1, la dystrophie rétinienne étant sélectionnée dans le groupe consistant en les suivantes : rétinite pigmentaire (RP) ; glaucome ; ou dégénérescence maculaire liée à l'âge (DMLA).

3. Composé selon la revendication 1 ou 2 pour l'utilisation de la revendication 1 ou 2, ledit composé étant la 13β,17α-diéthyl-17β-hydroxygon-4-èn-3-one ou un sel pharmaceutiquement acceptable de celle-ci.

4. Composé selon l'une quelconque des revendications précédentes pour une utilisation de l'une quelconque des revendications précédentes, ledit composé étant administré localement dans l'oeil.

5. Composé selon la revendication 4 pour l'utilisation de la revendication 4, ledit composé étant administré par instillation d'un collyre, en injection intravitréenne ou en injection intraoculaire.

6. Composition pharmaceutique qui est formulée sous la forme d'une solution pour une utilisation dans le traitement d'une dystrophie rétinienne et se prête à une administration locale dans l'oeil par injection intraoculaire et/ou injection intravitréenne, la composition comprenant un véhicule pharmaceutiquement acceptable et un composé qui est une 13-polycarbon-alkyl-17-alkyl-17β-hydroxygon-4-èn-3-one, ledit composé étant la 13β,17α-diéthyl-17β-hydroxygon-4-èn-3-one.
